# EUROPEAN PATENT APPLICATION

(11) **EP 3 928 805 A1**
(43) Date of publication of application: **29.12.2021**
(21) Application number: 20182683.1
(22) Date of filing: 26.06.2020
(51) Int. Cl.: A61L 27/18, A61L 27/20, A61L 27/52, A61L 27/54, A61L 31/04, A61L 31/06, A61L 31/14, A61L 31/16

(54) **A DEVICE FOR USE IN THE DELIVERY OF AN ACTIVE AGENT**

(71) Applicant: Royal College of Surgeons in Ireland, Dublin 2 (IE); University College Dublin, National University of Ireland, Dublin, Dublin 4 (IE)
(72) Inventor: HIBBITTS, Alan, Dublin, 2 (IE); LEMOINE, Riffington, Dublin, 2 (IE); O'BRIEN, Fergal, Dublin, 2 (IE); O'BRIEN, Colm, Dublin, 4 (IE)
(74) Representative: FRKelly

(57) **Abstract**

The present invention relates to a device for use in the delivery of an active agent. Specifically, the present invention relates to a device comprising a scaffold that can be formed from a polymer or copolymer; a matrix that can be formed from hyaluronic acid; and at least one active agent. The device finds utility in the delivery of at least one active agent, and is useful in methods of reducing collagen deposition in a tissue or treating fibrosis in a tissue. In an aspect, the device can alleviate postoperative inflammation and/or fibrosis; and can also improve patient compliance for effective delivery of an active agent.

## Description

### Field of the Invention

The present invention relates to a device for use in the delivery of an active agent. Specifically, the present invention relates to a device comprising a scaffold that can be formed from a polymer or co-polymer; a matrix that can be formed from hyaluronic acid; and at least one active agent. The device finds utility in the delivery of at least one active agent, and is useful in methods of reducing collagen deposition in a tissue or treating fibrosis in a tissue.

### Background to the Invention

Glaucoma results from the degeneration of the axons of retinal ganglion cells (RGCs), which make up the optic nerve. The degeneration of the RGCs leads to loss of vision and, if untreated or ineffectively treated, ultimately can lead to blindness. Approximately 67 million people suffer from glaucoma globally and, due to ineffective treatment, glaucoma is the second leading cause of irreversible blindness.

The mainstay of glaucoma treatment includes active agents that act to lower intraocular pressure (IOP). However, with the incidence of glaucoma increasing with age, poor patient compliance is a fundamental problem, whereby approximately 20% of patients eventually require surgery due to ineffective delivery of active agents.

Of these surgical interventions, the majority take the form of a surgical procedure known as a "trabeculectomy" or the insertion of a tubular drainage device. In both cases, the desired goal is to allow for fluid to flow from the interior to the exterior of the eye and thus reduce IOP. However, success rates for these interventions are marred by the body's own response to the surgery (such as inflammation, often followed by conjunctival scarring and/or fibrosis) and are further compounded by poor patient compliance in the use of prescribed anti-inflammatory eye-drops in the post-operative period.

Thus, there is a need for a device for use in the delivery of an active agent which can alleviate post-operative inflammation and/or fibrosis; and which can also improve patient compliance for effective delivery of the active agent.

### Summary of the Invention

According to a first aspect of the present invention, there is provided a device comprising:
(a) a scaffold, optionally formed from a polymer or co-polymer;
(b) a matrix, optionally formed from hyaluronic acid; and
(c) at least one active agent.

Optionally, the scaffold is formed from a biodegradable polymer or co-polymer.

Optionally, the scaffold is formed from a polyester.

Optionally, the scaffold is formed from a biodegradable polyester.

Optionally, the scaffold is formed from a thermoplastic polyester. Further optionally, the scaffold is formed from a thermoplastic biodegradable polyester.

Optionally, the scaffold is formed from polycaprolactone (1,7-Polyoxepan-2-one; Poly(hexano-6-lactone)). Alternatively, the scaffold is formed from polylactic acid (polylactide).

Preferably, the scaffold is formed from polycaprolactone (1,7-Polyoxepan-2-one; Poly(hexano-6-lactone)).

Optionally, the scaffold is formed from a biodegradable polymer or co-polymer selected from polysaccharides such as starch, chitosan, alginate, or hyaluronic acid; polyhydroxyalkanoates such as polyhydroxybutyrate; polylactic acid (polylactide); poly(lactic-co-glycolic acid); polyglycolide; poly(2-hydroxyethyl-methacrylate); poly(ethylene glycol); and polypeptides such as collagen or gelatine.

Optionally, the scaffold has a substantially trapezoidal shape. Further optionally, the scaffold has a substantially trapezoidal cross-section. Still further optionally, the scaffold has a substantially frustoconical shape.

Preferably, the scaffold has a substantially frustoconical shape.

Optionally, the scaffold comprises at least one layer. Further optionally, the scaffold comprises a plurality of layers.

Optionally, the or each layer has a thickness of at least 0.10 mm, optionally at least 0.12 mm, optionally at least 0.14 mm, optionally at least 0.16 mm, optionally at least 0.18 mm, optionally at least 0.20 mm. Further optionally, the scaffold comprises a plurality of layers, wherein each layer has a thickness of at least 0.10 mm, optionally at least 0.12 mm, optionally at least 0.14 mm, optionally at least 0.16 mm, optionally at least 0.18 mm, optionally at least 0.20 mm.

Preferably, the or each layer has a thickness of at least 0.16 mm. Further preferably, the scaffold comprises a plurality of layers, wherein each layer has a thickness of at least 0.16 mm.

Optionally, the or each layer comprises a peripheral member.

Optionally the peripheral member is substantially elliptical in shape. Further optionally, the peripheral member is substantially circular in shape.

Preferably, the peripheral member is substantially circular in shape.

Optionally, the peripheral member is substantially circular in shape and comprises at least one point of discontinuity. Optionally, the peripheral member is substantially annular in shape and comprises at least one point of discontinuity.

Optionally, the peripheral member defines a least one aperture.

Optionally, the peripheral member is substantially annular in shape. Further optionally, the peripheral member is substantially annular in shape and defines at least one aperture.

Optionally, the or each layer further comprises at least one cross-bar.

Optionally, the or each cross-bar is substantially linear in shape.

Optionally, the or each layer comprises a peripheral member and at least one cross-bar.

Optionally, the or each layer comprises a peripheral member which is substantially annular in shape and defines at least one aperture, and at least one cross-bar. Further optionally, the or each layer comprises a peripheral member which is substantially annular in shape and defines at least one aperture, and at least one cross-bar spanning the aperture. Still further optionally, the or each layer comprises a peripheral member which is substantially annular in shape and defines at least one aperture, and at least one cross-bar radially spanning the aperture. Still further optionally, the or each layer comprises a peripheral member which is substantially annular in shape and defines at least one aperture, and at least one cross-bar extending diametrically across the aperture.

Preferably, the or each layer comprises a peripheral member which is substantially annular in shape and defines at least one aperture, and at least one cross-bar extending diametrically across the aperture.

Optionally, the scaffold comprises at least two layers. Further optionally, the scaffold comprises a plurality of layers. Still further optionally, the scaffold comprises a plurality of contiguous layers. Still further optionally, the scaffold comprises a plurality of contiguous adjacent layers.

Optionally, the diameter of each layer is the same as the diameter of each adjacent layer. Alternatively, the diameter of each layer is different to the diameter of each adjacent layer. Optionally, the diameter of each layer is less than the diameter of each adjacent layer. Further optionally, the diameter of each layer is less than the diameter of each subsequent adjacent layer.

Optionally, the matrix is formed from a biodegradable polymer or co-polymer.

Optionally, the matrix is formed from a biodegradable polysaccharide.

Optionally, the matrix is formed from hyaluronic acid. Further optionally, the matrix is formed from hyaluronic acid solution.

Optionally, the hyaluronic acid solution is a 0.1 - 2.0%(w/v) hyaluronic acid solution, optionally a 0.1 - 1.5%(w/v) hyaluronic acid solution, optionally a 0.1 - 1.0%(w/v) hyaluronic acid solution, optionally a 0.25 - 0.75%(w/v) hyaluronic acid solution, optionally a 0.5%(w/v) hyaluronic acid solution.

Optionally, the hyaluronic acid solution is a 0.1 - 2.0%(w/v) hyaluronic acid aqueous solution, optionally a 0.1 - 1.5%(w/v) hyaluronic acid aqueous solution, optionally a 0.1 - 1.0%(w/v) hyaluronic acid aqueous solution, optionally a 0.25 - 0.75%(w/v) hyaluronic acid aqueous solution, optionally a 0.5%(w/v) hyaluronic acid aqueous solution.

Preferably, the hyaluronic acid solution is a 0.5%(w/v) hyaluronic acid aqueous solution.

Optionally, the matrix is a cross-linked matrix.

Optionally, the matrix is a cross-linked hyaluronic acid matrix. Further optionally, the matrix is a cross-linked hyaluronic acid solution matrix.

Preferably, the matrix is a cross-linked hyaluronic acid solution matrix.

Optionally, the matrix has a pH of less than 7.0, optionally a pH of less than 6.0, optionally, a pH of less than 5.5, optionally a pH of less than 5.0, optionally, a pH of less than 4.9, optionally, a pH of less than 4.8, optionally, a pH of less than 4.75, optionally, a pH of less than 4.7, optionally, a pH of less than 4.6, optionally, a pH of less than 4.5, optionally, a pH of less than 4.0, optionally, a pH of less than 3.5, optionally, a pH of less than 3.0.

Preferably, the matrix has a pH of less than 4.8. Further preferably, the matrix has a pH of 4.75.

Optionally, the at least one active agent is a protein. Further optionally, the at least one active agent is a polypeptide.

Optionally, the at least one active agent is a proteoglycan. Further optionally, the at least one active agent is a small leucine-rich proteoglycan. Still further optionally, the at least one active agent is decorin.

Alternatively, the at least one active agent is pirfenidone (5-Methyl-1-phenylpyridin-2-one).

Alternatively, the at least one active agent is a steroidal active agent. Further alternatively, the at least one active agent is a corticosteroidal active agent. Still further alternatively, the at least one active agent is a glucocorticoid active agent. Still further alternatively, the at least one active agent is prednisolone (11β-11,17,21-Trihydroxypregna-1,4-diene-3,20-dione).

Optionally, the scaffold comprises the at least one active agent. Further optionally, the scaffold comprises at least 0.01%(w/w) active agent, optionally at least 0.25%(w/w) active agent, optionally at least 0.5%(w/w) active agent, optionally at least 1.0%(w/w) active agent, optionally at least 1.5%(w/w) active agent, optionally at least 2.0%(w/w) active agent, optionally at least 2.5%(w/w) active agent, optionally at least 3.0%(w/w) active agent, optionally at least 3.5%(w/w) active agent.

Preferably, the scaffold comprises at least 3.0%(w/w) active agent.

Optionally or additionally, the matrix comprises the at least one active agent. Further optionally or additionally, the matrix comprises at least 0.01 %(w/w) active agent, optionally at least 0.25%(w/w) active agent, optionally at least 0.5%(w/w) active agent, optionally at least 1.0%(w/w) active agent, optionally at least 1.5%(w/w) active agent, optionally at least 2.0%(w/w) active agent, optionally at least 2.5%(w/w) active agent, optionally at least 3.0%(w/w) active agent, optionally at least 3.5%(w/w) active agent.

Preferably, the matrix comprises at least 3.0%(w/w) active agent.

Optionally, the scaffold and the matrix each comprise the at least one active agent. Further optionally, the scaffold and the matrix each comprise at least 0.01 %(w/w) active agent, optionally at least 0.25%(w/w) active agent, optionally at least 0.5%(w/w) active agent, optionally at least 1.0%(w/w) active agent, optionally at least 1.5%(w/w) active agent, optionally at least 2.0%(w/w) active agent, optionally at least 2.5%(w/w) active agent, optionally at least 3.0%(w/w) active agent, optionally at least 3.5%(w/w) active agent.

Preferably, the scaffold and the matrix each comprise at least 3.0%(w/w) active agent. Preferably, the scaffold and the matrix together comprise at least 6.0%(w/w) active agent.

Optionally, the scaffold and the matrix each comprise the same active agent. Alternatively, the scaffold and the matrix each comprise a different active agent.

Optionally, the scaffold is at least partially incorporated in the matrix. Further optionally, the scaffold is incorporated in the matrix.

Optionally or additionally, the matrix at least partially surrounds the scaffold. Further optionally or additionally, the matrix surrounds the scaffold.

According to a second aspect of the present invention there is provided a scaffold for use in a device according to the first aspect of the present invention.

Optionally, the scaffold is for use in the delivery of at least one active agent.

Optionally, there is provided a scaffold for use in the delivery of at least one active agent.

According to a third aspect of the present invention there is provided a matrix for use in a device according to the first aspect of the present invention.

Optionally, the matrix is for use in the delivery of at least one active agent.

Optionally, there is provided a matrix for use in the delivery of at least one active agent.

According to a fourth aspect of the present invention, there is provided a method of manufacturing a device according to the first aspect of the present invention, the method comprising the steps of
(i) providing a scaffold, optionally formed from a polyester;
(ii) providing a matrix, optionally formed from hyaluronic acid; and
(iii) providing an active agent.

Optionally, the providing a scaffold step comprises providing a scaffold formed from a polyester.

Optionally, the providing a scaffold step comprises providing a scaffold having a substantially trapezoidal shape. Further optionally, the providing a scaffold step comprises providing a scaffold having a substantially trapezoidal cross-section. Still further optionally, the providing a scaffold step comprises providing a scaffold having a substantially frustoconical shape.

Preferably, the providing a scaffold step comprises providing a scaffold having a substantially frustoconical shape.

Optionally, the providing a scaffold step comprises providing at least one layer of the scaffold. Further optionally, the providing a scaffold step comprises providing a plurality of layers of the scaffold.

Optionally, the providing a scaffold step comprises depositing at least one layer of the scaffold. Further optionally, the providing a scaffold step comprises depositing a plurality of layers of the scaffold.

Optionally, the providing a scaffold step comprises sequentially depositing at least one layer of the scaffold. Further optionally, the providing a scaffold step comprises sequentially depositing a plurality of layers of the scaffold.

Optionally, the providing a scaffold step comprises additively depositing at least one layer of the scaffold. Further optionally, the providing a scaffold step comprises additively depositing a plurality of layers of the scaffold.

Preferably, the providing a scaffold step comprises additively depositing a plurality of layers of the scaffold.

Optionally, the providing a scaffold step comprises depositing at least one layer of the scaffold through an outlet, optionally through an outlet having a wire gauge of 37, optionally a wire gauge of 36, optionally a wire gauge of 35, optionally a wire gauge of 34, optionally a wire gauge of 33, optionally a wire gauge of 32, optionally a wire gauge of 31, optionally a wire gauge of 30, optionally a wire gauge of 29, optionally a wire gauge of 28, optionally a wire gauge of 27, optionally a wire gauge of 26, optionally a wire gauge of 25.

Preferably, the providing a scaffold step comprises depositing at least one layer of the scaffold through an outlet having a wire gauge of 30, 27, or 25.

Optionally, the providing a scaffold step comprises depositing a plurality of layers of the scaffold through an outlet.

Preferably, the providing a scaffold step comprises depositing a plurality of layers of the scaffold through an outlet having a wire gauge of 30, 27, or 25.

Optionally, the providing a scaffold step comprises 3D-printing at least one layer of the scaffold. Further optionally, the providing a scaffold step comprises 3D-printing a plurality of layers of the scaffold.

Optionally, the providing a matrix step comprises providing a matrix formed from hyaluronic acid.

Optionally, the providing a matrix step comprises providing a hyaluronic acid solution.

Optionally, the hyaluronic acid solution is a 0.1 - 2.0%(w/v) hyaluronic acid solution, optionally a 0.1 - 1.5%(w/v) hyaluronic acid solution, optionally a 0.1 - 1.0%(w/v) hyaluronic acid solution, optionally a 0.25 - 0.75%(w/v) hyaluronic acid solution, optionally a 0.5%(w/v) hyaluronic acid solution.

Optionally, the hyaluronic acid solution is a 0.1 - 2.0%(w/v) hyaluronic acid aqueous solution, optionally a 0.1 - 1.5%(w/v) hyaluronic acid aqueous solution, optionally a 0.1 - 1.0%(w/v) hyaluronic acid aqueous solution, optionally a 0.25 - 0.75%(w/v) hyaluronic acid aqueous solution, optionally a 0.5%(w/v) hyaluronic acid aqueous solution.

Preferably, the hyaluronic acid solution is a 0.5%(w/v) hyaluronic acid aqueous solution.

Optionally, the providing a matrix step further comprises cross-linking the hyaluronic acid or the hyaluronic acid solution.

Preferably, the providing a matrix step further comprises cross-linking the hyaluronic acid solution.

Optionally, the cross-linking step comprises adding at least one cross-linking agent.

Optionally, the at least one cross-linking agent is a dihydrazine compound. Further optionally, the at least one cross-linking agent is adipic dihydrazide (ADH; hexanedihydrazide).

Optionally, the at least one cross-linking agent is at least 0.63 mmol of a dihydrazine compound. Further optionally, the at least one cross-linking agent is at least 0.63 mmol of adipic dihydrazide (ADH; hexanedihydrazide).

Optionally, the at least one cross-linking agent is a carbodiimide compound. Further optionally, the at least one cross-linking agent is 1-Ethyl-3-(3-dimethylaminopropyl)carbodiimide (EDC; EDAC; EDCI; 3-(Ethyliminomethyleneamino)-N,N-dimethylpropan-1-amine).

Optionally, the at least one cross-linking agent is at least 52 mmol of a carbodiimide compound. Further optionally, the at least one cross-linking agent is at least 52 mmol of 1-Ethyl-3-(3-dimethylaminopropyl)carbodiimide (EDC; EDAC; EDCI; 3-(Ethyliminomethyleneamino)-N,N-dimethylpropan-1-amine).

Optionally, the providing a matrix step comprises adjusting the pH of the matrix to a pH of less than 7.0, optionally a pH of less than 6.0, optionally, a pH of less than 5.5, optionally a pH of less than 5.0, optionally, a pH of less than 4.9, optionally, a pH of less than 4.8, optionally, a pH of less than 4.75, optionally, a pH of less than 4.7, optionally, a pH of less than 4.6, optionally, a pH of less than 4.5, optionally, a pH of less than 4.0, optionally, a pH of less than 3.5, optionally, a pH of less than 3.0, Preferably, the providing a matrix step comprises adjusting the pH of the matrix to a pH of less than 4.8. Further preferably, the providing a matrix step comprises adjusting the pH of the matrix to a pH of 4.75.

Optionally, the adjusting step comprises adding an acid, optionally adding hydrochloric acid.

Optionally, the adjusting step comprises adding 0.1 - 1 M acid, optionally, 0.1 - 0.5M acid, optionally 0.1 M acid. Optionally, the adjusting step comprises adding 0.1 - 1M hydrochloric acid, optionally, 0.1 - 0.5M hydrochloric acid, optionally 0.1 M hydrochloric acid.

Optionally, the cross-linking step further comprises agitating the matrix. Further optionally, the cross-linking step further comprises stirring the matrix.

Optionally, the agitating step is conducted for at least 1 hour, optionally at least 2 hours, optionally at least 4 hours, optionally at least 8 hours, optionally at least 12 hours, optionally at least 24 hours.

Optionally, the providing a matrix step comprises purifying the matrix.

Optionally, the purifying step comprises at least one dialysis treatment of the matrix. Further optionally, the purifying step comprises at least one dialysis treatment of the hyaluronic acid. Still further optionally, the purifying step comprises at least one dialysis treatment of the hyaluronic acid solution.

Optionally, the at least one dialysis treatment is selected from dialysing against a salt or salt solution, an alcohol or alcohol solution, and an aqueous solution. Further optionally, the at least one dialysis treatment is selected from dialysing against sodium chloride or a sodium chloride solution, ethanol or an ethanol solution, and an aqueous solution. Still further optionally, the at least one dialysis treatment is selected from dialysing against 100mM sodium chloride solution, 20%(v/v) ethanol solution, and an aqueous solution.

Optionally, the or each dialysis treatment is conducted fro at least 12 hours, optionally at least 24 hours, optionally at least 48 hours.

Optionally, the providing the active agent step comprises incorporating the active agent into the matrix.

Optionally, the providing the active agent step comprises incorporating the active agent into the matrix and agitating the matrix, optionally stirring the matrix.

Optionally, the providing the active agent step comprises adding the active agent into the matrix. Optionally, the providing the active agent step comprises adding the active agent into the matrix and agitating the matrix, optionally stirring the matrix.

Optionally, the providing the active agent step comprises adding the active agent into the matrix with a co-polymer.

Optionally, the adding step comprises adding the active agent to the co-polymer. Further optionally, the adding step comprises adding the active agent to the co-polymer in the presence of a solvent.

Optionally, the co-polymer is poly(lactic-co-glycolic acid).

Optionally, the solvent is dichloromethane (DCM; methylene chloride).

Optionally, the adding step further comprises adding the active agent and the co-polymer (and optionally the solvent) to a vinyl polymer to form nanoparticles.

Optionally, the vinyl polymer is poly(vinyl alcohol).

Optionally, the adding step further comprises dehydrating the nanoparticles. Further optionally, the adding step further comprises drying the nanoparticles. Still further optionally, the adding step further comprises freeze-drying the nanoparticles. Still further optionally, the adding step further comprises lyophilising the nanoparticles.

Optionally, the providing the active agent step comprises incorporating the active agent into the scaffold.

Optionally, the providing the active agent step comprises adding the active agent into the scaffold.

Optionally, the adding step comprises adding the active agent to the scaffold in the presence of a solvent to form an admixture.

Optionally, the solvent is dichloromethane (DCM; methylene chloride).

Optionally, the adding step further comprises dehydrating the admixture. Further optionally, the adding step further comprises drying the admixture.

Optionally, the adding step is conducted prior to the step of providing at least one layer of the scaffold, optionally prior to the step of providing a plurality of layers of the scaffold.

According to a fifth aspect of the present invention there is provided a method of manufacturing a scaffold for use in a device according to the first aspect of the present invention.

Optionally, the scaffold is for use in the delivery of at least one active agent.

Optionally, there is provided a method of manufacturing a scaffold for use in the delivery of at least one active agent.

According to a sixth aspect of the present invention there is provided a method of manufacturing a matrix for use in a device according to the first aspect of the present invention.

Optionally, the matrix is for use in the delivery of at least one active agent.

Optionally, there is provided a method of manufacturing a matrix for use in the delivery of at least one active agent.

According to a seventh aspect of the present invention, there is provided a method for the delivery of at least one active agent, the method comprising the step of incorporating the at least one active agent into a scaffold according to the second aspect of the present invention or a scaffold manufactured by the method according to the fifth aspect of the present invention.

Optionally, the method comprises the step of applying a device comprising the scaffold. Further optionally, the method comprises the step of topically applying a device comprising the scaffold.

According to an eighth aspect of the present invention, there is provided a method for the delivery of at least one active agent, the method comprising the step of incorporating the at least one active agent into a matrix according to the third aspect of the present invention or a matrix manufactured by the method according to the sixth aspect of the present invention.

Optionally, the method comprises the step of applying a device comprising the matrix. Further optionally, the method comprises the step of topically applying a device comprising the matrix.

According to a further aspect of the present invention, there is provided a method of reducing inflammation and/or collagen deposition in a tissue, the method comprising the step of applying a device according to a first aspect of the present invention to the tissue.

According to a further aspect of the present invention, there is provided a method of treating fibrosis in a tissue, the method comprising the step of applying a device according to a first aspect of the present invention to the tissue.

According to a further aspect of the present invention, there is provided a method of treating glaucoma in a tissue, the method comprising the step of applying a device according to a first aspect of the present invention to the tissue.

### Brief Description of the Drawings

Embodiments of the present invention will now be described with reference to the appended nonlimiting examples and the accompanying drawings, in which:
Figure 1 illustrates the effect of matrix hyaluronic acid content on gel hydration;
Figure 2 illustrates the effect of matrix pH at the time of crosslinking on final gel hydration (top) and resistance to enzymatic degradation (bottom);
Figure 3 illustrates (A) a planar view of a multi-diameter mould used to form and freeze-dry the device; wherein diameters or each layer of the scaffold were varied, but the mould height was constant at 5 mm; and (B) a schematic overview of the method of manufacture of the device, wherein matrix hydrogel crosslinking and scaffold 3D printing can be conducted in parallel and the matrix and scaffolds combined to form the final device;
Figure 4 illustrates the anti-fibrotic potential of decorin, wherein primary human conjunctival fibroblasts were treated with the pro-inflammatory molecule TGF-β +/- decorin doses and the change in collagen deposition was assessed;
Figure 5 illustrates Python program derived images of 3D printed tapered polymer scaffolds, wherein (A) depicts a perspective view of a polymer scaffold during the layer deposition (print) process; (B) depicts a perspective view of a fully formed polymer scaffold; (C) depicts a plan view of a fully formed polymer scaffold; and (D) - (G) depict plan views of different embodiments of fully formed polymer scaffolds;
Figure 6 illustrates encapsulation of pirfenidone in PLGA nanoparticles using scanning electron microscopy (SEM), wherein images from left depicts scanning electron micrograph of PLGA particles alone; low; and high magnification images of PLGA-pirfenidone nanoparticles dispersed in a freeze-dried device;
Figure 7 depicts a scanning electron micrograph of 25kDa (left) and prednisolone loaded PCL (right) scaffolds, wherein the higher magnification images (bottom) illustrate deposition of prednisolone visible on the struts;
Figure 8 illustrates direct incorporation of pirfenidone into 25kDa PCL scaffolds via 3D printing using 1% w/w and 3% w/w samples, wherein (A) depicts daily release and (B) depicts cumulative release rates; (C) depicts further comparison of 1% w/w scaffold alone, scaffold in a freeze dried HyA matrix and non-printed PCL-pirfenidone;
Figure 9 illustrates the effect of different molecular weights of PCL for drug printing when combined with the hyaluronic acid matrix, wherein (A) depicts a lower molecular weight PCL where release was slowed when added to the matrix and (B) depicts use of a higher (50kDa) molecular weight PCL where there was a negligible difference in the presence of the hydrated matrix;
Figure 10 illustrates daily (left) and cumulative (right) 3D printed prednisolone drug release which demonstrated biphasic release up to 28 days;
Figure 11 depicts daily prednisolone release from nanoparticles in a freeze-dried scaffold and hyaluronic acid matrix; and (right) depicts cumulative rapid and sustained release; and
Figure 12 illustrates prednisolone loaded PCL meshes demonstrated a dose dependent ability to inhibit angiogenesis in chick embryos at day 5 post implantation.

### Examples

### Example 1

### Providing a matrix

A matrix was provided as a hyaluronic acid hydrogel formed via a crosslinking reaction. Briefly, 0.5%(w/v) hyaluronic acid (HA) (Contipro, Dolní Dobrouč̌̌̌, Czech Republic. Molecular weight 1.5 MDa) was dissolved in deionised water. This was followed by the addition of 0.63 mmol of adipic dihydrazide (ADH) (Sigma-Aldrich, Ireland) and 52 mmol of 1-Ethyl-3-[3-(dimethylamino)-propyl]carbodiimide EDAC) (Sigma-Aldrich, Ireland). The pH of the solution was decreased to pH 4.75 by dropwise addition of 0.1M hydrochloric acid (HCI) and the reaction was left under mild stirring for 24 hours (Scheme 1).

Following crosslinking, the solution was purified by successive dialysis using cellulose membrane dialysis tubing having a molecular-weight cut-off of 14,000 Da (Sigma-Aldrich, Ireland) against 100mM NaCI for 48 hours, 20%(v/v) ethanol for 24 hours and deionised water for 24 hours. At that point, the crosslinked matrix was stored at 4°C until required.

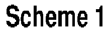

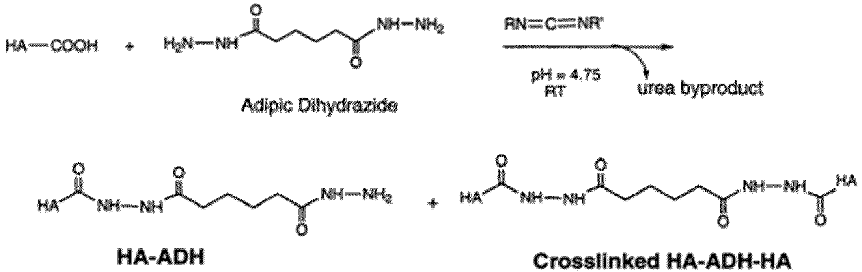

Scheme 1 illustrates the matrix crosslinking reaction, wherein the carboxyl (-COOH) group of the HA was reacted with the bridging ligand ADH (Sigma-Aldrich, Ireland) at low pH and in the presence of EDAC to produce the final crosslinked matrix.

### Example 2

### Effect of matrix hyaluronic acid content

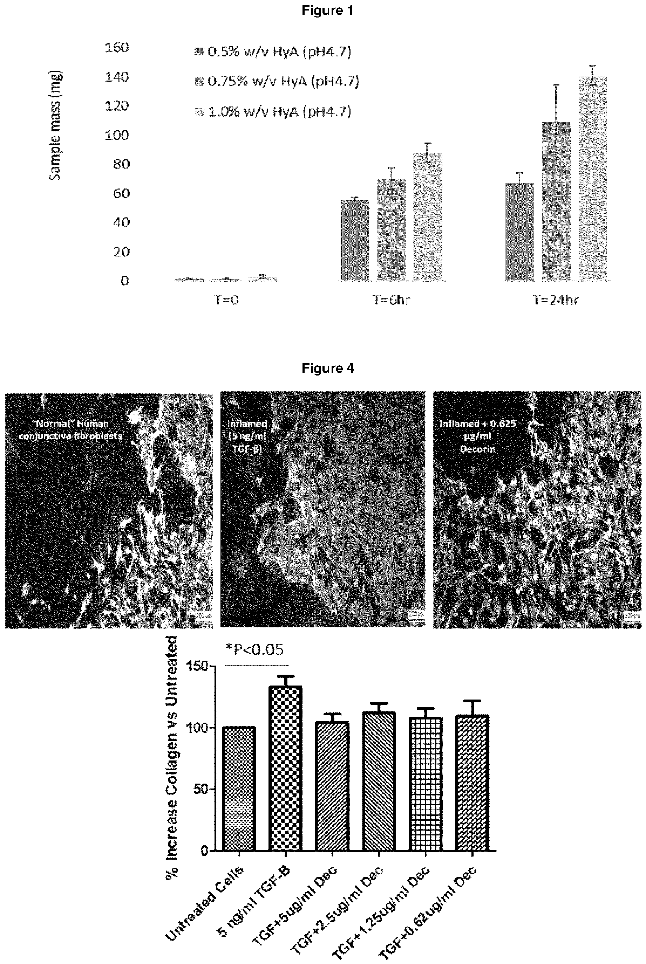

In order to ensure the cross-linked matrix would be capable of rapid rehydration, crosslinking was undertaken as in Example 1 using three different matrix HA concentrations - 0.5 %(w/v), 0.75%(w/v), and 1.0%(w/v). Following freeze-drying, three matrix samples, each having an 8mm diameter x 5mm height, from each concentration group were weighed separately and placed in phosphate buffered saline (PBS, pH 7.4) at 37°C to rehydrate. At 30 minutes, 6 hours, and 24 hours post-hydration, matrix samples were removed from PBS and gently dabbed on pre-moistened blotting paper to remove excess PBS. Samples were then re-weighed before being returned to PBS for the next time point or discarded after 24 hours (Figure 1).

### Example 3

### Effect of matrix pH

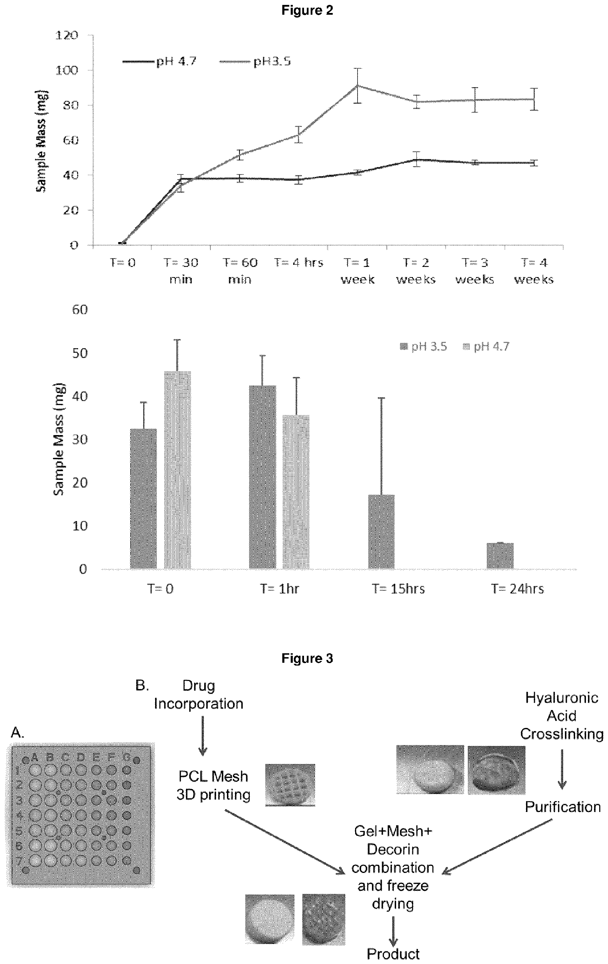

Matrix hydrogels were formed at 0.5%(w/v) as described in Example 1 with reaction pH lowered to either pH 3.5 or pH 4.75. Following freeze-drying, three matrix samples from each pH group were weighed separately and allowed to rehydrate in PBS as described in Example 3. At selected time points of 30 min, 60 min, 4 hr, 1 weeks, 2 weeks, 3 weeks, and 4 weeks, samples were dried on pre-moistened blotting paper and changes in mass were recorded. Readings were taken up to four weeks post-hydration (Figure 2, Top).

In addition to changes in hydration rate and stability, matrix gels crosslinked at either pH 4.75 or pH 3.5 were examined for differences in degradation rate in the presence of a hyaluronidase enzyme (Sigma-Aldrich, Ireland). Following hydration in PBS as described in Example 3, samples were transferred to 1 ml of PBS containing 180 IU of hyaluronidase enzyme and incubated at 37°C. At selected time points of 1hr, 15 hrs, and 24 hrs samples were dried on pre-moistened blotting paper and changes in mass were recorded (Figure 2, bottom).

### Example 4

### Providing a device

Following preparation of an active-agent-incorporated-matrix as described in Example 6, and the preparation of an active-agent-incorporated-scaffold as described in Example 6, the scaffold was incorporated into the matrix to form a device, and the device was freeze-dried under controlled conditions. Specifically, a variable diameter stainless steel mould was used to allow for control over the final dimensions of the device (see Figure 3A). The mould was first filled with a degassed and cross-linked hyaluronic acid (from Example 1), without and with active agent (from Example 5) either freely dispersed or in the form of active-agent-encapsulated nanoparticles. Following this, a scaffold without active agent (from Example 6) or with active agent (from Example 7) was inserted into the matrix and allowed to sit for 5 minutes to allow for air pockets to be identified. Any gas pockets caused by scaffold insertion were worked free by either gently moving the scaffold or manually extracting gas with a small volume micro-pipette. Finally, the mould was placed on a freeze dryer shelf and freeze dried under controlled conditions. Typically, this was a 1°C/min temperature decrease to a final temperature of -40°C with drying taking place over 25 hours, thus creating a micro-porous structure in the device. Freeze-dried devices were then recovered and stored at 2-8°C for further use. The overall production process is described in Figure 3B.

### Example 5

### Validation of a naturally anti-fibrotic active agent

The ability of the active agent decorin to inhibit collagen deposition was assessed *in vitro* using primary human conjunctival fibroblasts (obtained from Innoprot, Spain). Cells were seeded at a density of 0.25 x 10e4 cells per well in a 96-well tissue culture plate 24 hours prior to experiments. On the day of the experiment, with the exception of the negative control, all cells then received media that had been supplemented with 5 ng/ml of the pro-inflammatory molecule TGF-β (ProSpec-Tany TechnoGene Ltd, Israel) to induce fibrosis/collagen deposition. In addition to that, cell culture media of test wells was further augmented with concentrations of decorin ranging from 10 µg/ml to 0.625 µg/ml. Cells were cultured under standard conditions for 24 hours prior to fixation with chilled methanol overnight at -20°C. Samples were then stained for collagen deposition using 0.1 %(w/v) pico-sirius red for 4 hours. Samples were first imaged under a light microscope and the stain was then incubated in 200 µL 0.1 M sodium hydroxide and the plate was rocked on a rocking platform for 2 hours to elute the stain. Optical density was determined using a spectrophotometer at 540 nm (Figure 4).

### Example 6

### Providing a scaffold

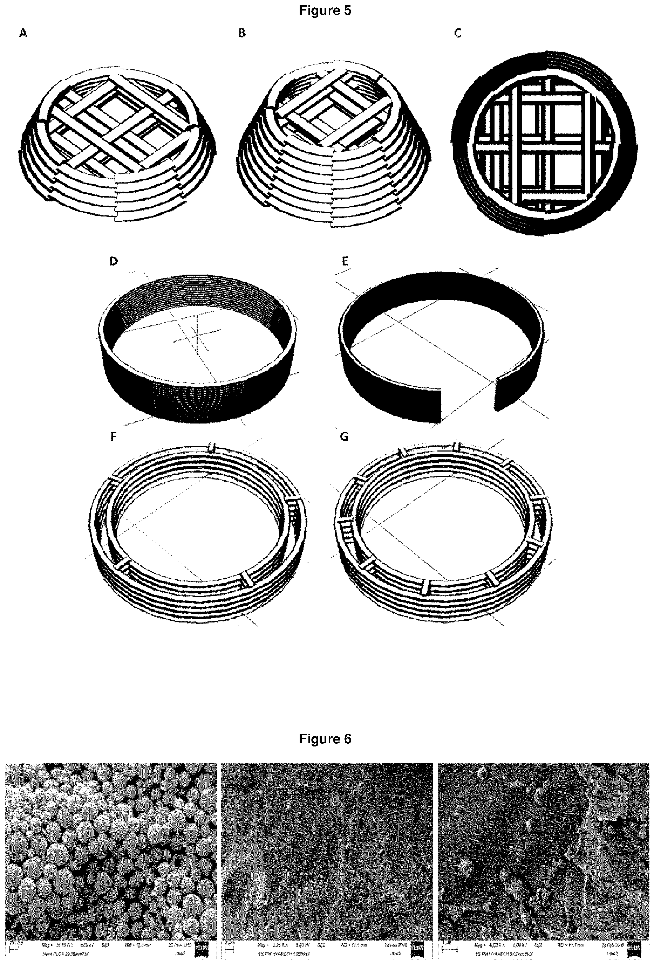

An Allevi2 bioprinter was used to 3D print various molecular weights of the scaffold polymer (typically, polycaprolactone (PCL) (Polysciences Europe GmbH, Germany) using a 30-gauge needle with an inner diameter of 0.16 mm to produce thin fibres of PCL for layer by layer deposition. A custom-made Python program was used to generate the toolpaths for the 3D printed scaffold designs (see Figure 5). The 3D printed polymer scaffolds were designed to be tapered to reduce tearing/damage of tissue at the implantation/application site. The tapered structure was achieved by reducing the diameter of the circumference of each subsequent deposited layer as the height of the scaffold increased. The spacing of cross-bars in the form of parallel fibres of PCL in the mesh interior was kept constant at 2 mm for all layers. The height, diameter, and slope of the 3D printed tapered polymer scaffolds can be adjusted to suit different implantation sites.

### Example 7

### Active agent matrix and scaffold incorporation

Active agent was incorporated into the matrix of Example 1. Briefly, immediately prior to controlled freeze drying of a 1°C/min temperature decrease to a final temperature of -40°C with drying taking place over 25 hours; the desired amount of decorin (Sigma-Aldrich, Ireland) (typically 0.62 µg - 10 µg/ml) was added to the matrix and dispersed via vigorous stirring.

For sustained release, the active agent pirfenidone (LGM Pharma, USA) was encapsulated in poly(lactic-co-glycolic acid) (PLGA) (Sigma-Aldrich, Ireland) nanoparticles and dispersed in the matrix and then freeze-dried by a 1°C/min temperature decrease to a final temperature of -40°C with drying taking place over 25 hours. For the preparation of active agent and PLGA nanoparticles, a single emulsion approach was used, whereby 1%(w/w) active agent and PLGA were dissolved in a suitable organic solvent of dichloromethane (DCM) (Sigma-Aldrich, Ireland). This solution was then added drop-wise to an aqueous solution of 1% (w/w) polyvinyl alcohol (PVA) under sonication, resulting in the formation of colloidally-stable nanoparticles. Dispersions of nanoparticles were left under extractor fans overnight to evaporate any traces of solvent/DCM and pirfenidone-PLGA particle dispersions were snap-frozen in liquid nitrogen and transferred to a freeze dryer where they were dried overnight at minimum temperature of -40°C and 10 mbar pressure to yield the finalised active agent encapsulated in PLGA particle powders (Figure 6, left). These were then dispersed in the hyaluronic matrix prior to freeze drying of the final device (Figure 6, middle-right).

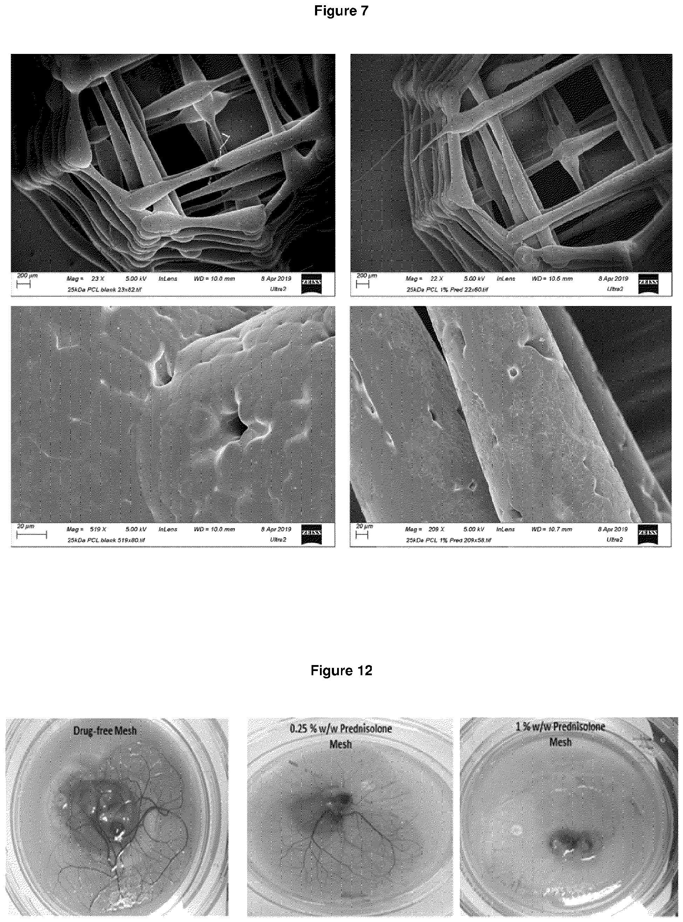

A more streamlined approach for the creation of a biphasic release profile of active agent was also established, whereby pirfenidone (LGM Pharma) or prednisolone (Sigma-Aldrich) was incorporated into the polymer scaffold prior to deposition/printing. Specifically, 0.25 - 3% (w/w) of active agent and PCL were dissolved in a suitable organic solvent (DCM) and left under a fume hood overnight to evaporate all DCM. The now re-solidified active-agent-incorporated-PCL was then loaded into the injector syringe of the 3D printer and scaffolds were printed as described in Example 5 (Figure 7).

### Example 8

### Active agent release

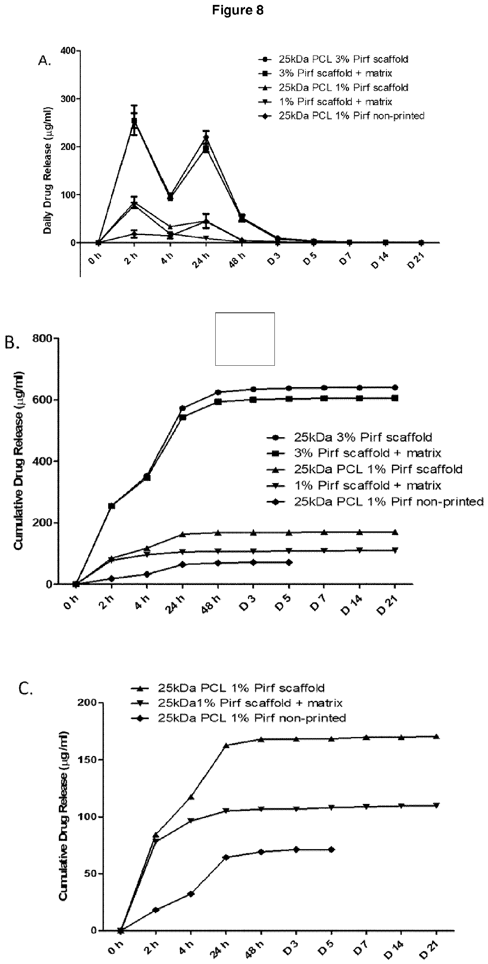

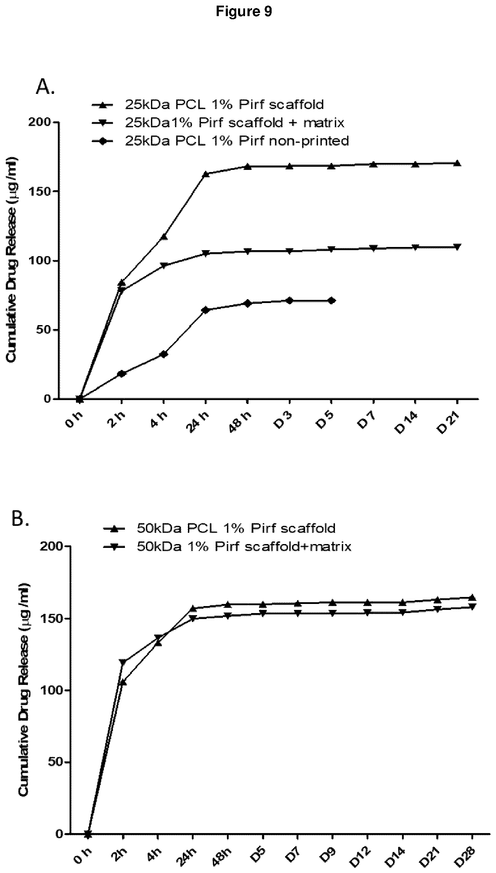

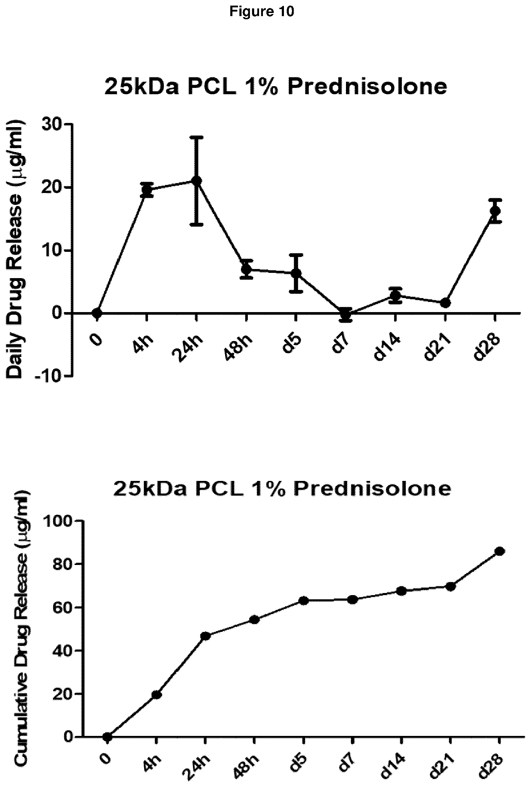

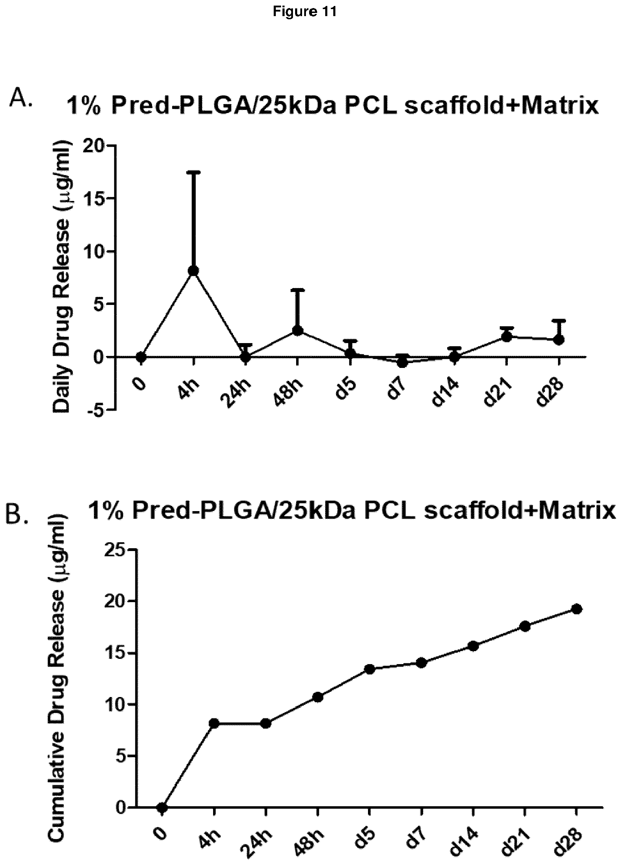

Release of nanoparticle-encapsulated active agent from a matrix and release of active agent from a scaffold was assessed in the same manner. In short, one sample (of either a printed mesh (scaffold) or mesh+gel (scaffold and matrix)) per 1.5 mL eppendorf tube was incubated in 1mL of PBS at 37°C under agitation in a heated water bath. The release buffer was completely exchanged at selected time points of T=4hrs, T=24 hrs, T=48 hrs, T=1 week, T=2 weeks, T=3 weeks, and T=4 weeks with samples stored at -20°C until analysis. Release studies were undertaken for up to 28 days and released active agent concentrations were determined via extrapolation from a standard curve. Blank scaffolds (containing no active agent) were tested and readings were subtracted as an additional background control. Samples containing pirfenidone were analysed at 317 nm and samples containing prednisolone were analysed at 245 nm. Using either 3D drug printing or nanoparticle incorporation, it was possible to achieve a rapid release of active agent in the first 24 hours as well as a continuous release of drug over a 28 day period without the need for separate loading strategies. Furthermore, the use of 3D printing resulted in release rates entirely different to unprinted polymers (Figure 8) which was also found to be reliant on the molecular weight of the printed scaffold (Figure 9). Choice of drug and means of drug encapsulation also resulted in marked differences in release kinetics (Figure 10+11).

### Example 9

### Effect of device active agent release

The effect of active agent release on angiogenesis over the immediate 5 days following device implantation/application was examined using the Chick Chorioallantoic Membrane (CAM) assay. The assay was set up as previously described by Ryan et al. In short, fertilised chicken eggs (day 0 of development) were supplied by Ovagen (Ovagen Group Ltd, Co. Mayo, Ireland). On receipt, the eggs were incubated for 3 days (until day 3 of development) lying in a horizontal position in a specialised incubator at 37 °C in regular atmospheric conditions. The eggs were turned every 24 hours for correct embryo orientation during development and for optimum CAM development. On day 3, the eggs were cracked into 100 mm Ø petri dishes (Corning Inc., New York, USA) and the lid was replaced. To keep the embryos humidified, the petri dish containing the embryo was placed into a larger 150 mm Ø petri dish (Corning Inc., New York, USA) containing 25 ml of sterile PBS and the lid of the larger petri dish was also replaced and the chick embryos were placed back in the incubator.

After a further 4 days of incubation (until day 7 of development), hydrated devices comprising a scaffold and matrix, or devices comprising only a scaffold (with no matrix) were applied topically to the CAM membrane. Furthermore, active-agent-free controls were also included as a test group, with all groups then returned to the incubator for a further 5 days (equal to day 12 of embryonic development). At that point, changes in blood vessel growth were assessed visually and recorded using a 13 MP digital camera (Samsung) (Figure 12). Embryos were destroyed using a combination of 38% paraformaldehyde and physical agitation prior to disposal. All experimentation carried out on chick embryos was in accordance with the EU Directive 2010/63/EU for animal experiments.

## Claims

1. A device comprising:
(a) a scaffold formed from polycaprolactone;
(b) a matrix formed from hyaluronic acid; and
(c) at least one active agent.

2. A device according to Claim 1, wherein the scaffold has a substantially frustoconical shape.

3. A device according to Claim 1 or 2, wherein the scaffold comprises a plurality of layers.

4. A device according to Claim 3, wherein the or each layer comprises a peripheral member which is substantially annular in shape and defines at least one aperture, and at least one cross-bar extending diametrically across the aperture.

5. A device according to any preceding claim, wherein the matrix is formed from a 0.1 - 2.0%(w/v) hyaluronic acid solution.

6. A device according to any preceding claim, wherein the matrix is a cross-linked matrix.

7. A device according to any preceding claim, wherein the matrix has a pH of less than 7.0.

8. A device according to any preceding claim, wherein the scaffold comprises at least 0.25%(w/w) of the active agent.

9. A device according to any preceding claim, wherein the matrix comprises at least 0.01 %(w/w) of the active agent.

10. A method of manufacturing a device according to the first aspect of the present invention, the method comprising the steps of
(a) providing a scaffold formed from polycaprolactone;
(b) providing a matrix formed from hyaluronic acid; and
(c) providing at least one active agent.

11. A method according to Claim 10, wherein the providing a scaffold step (a) comprises additively depositing a plurality of layers of the scaffold.

12. A method according to Claim 10 or 11, wherein providing at least one active agent step (c) comprises incorporating the or each active agent into the matrix.

13. A method according to any one of Claims 10-12 comprising adding the or each active agent and a co-polymer to a vinyl polymer to form nanoparticles.

14. A method according to any one of Claims 10-13, wherein the providing at least one active agent step (c) comprises incorporating the or each active agent into the scaffold.

15. A method for the delivery of at least one active agent, the method comprising the steps of:
(a) incorporating the at least one active agent into a scaffold formed from polycaprolactone;
(b) incorporating the at least one active agent into a matrix formed from hyaluronic acid; and
(c) providing a device comprising the scaffold formed from polycaprolactone, the matrix formed from hyaluronic acid, and the at least one active agent.
